# EUROPÄISCHE PATENTSCHRIFT

(11) **EP 2 509 678 B1**
(45) Veröffentlichungstag und Bekanntmachung des Hinweises auf die Patenterteilung: **13.02.2019**
(21) Anmeldenummer: 10796276.3
(22) Anmeldetag: 10.12.2010
(51) Int. Cl.: A61N 1/05

(54) **NERVENPROTHESE UND VERFAHREN ZUR HERSTELLUNG EINER NERVENPROTHESE**
NEURAL PROSTHESIS AND METHOD FOR PRODUCING A NEURAL PROSTHESIS
PROTHÈSE NERVEUSE ET PROCÉDÉ DE FABRICATION D'UNE PROTHÈSE NERVEUSE

(30) Priorität: 11.12.2009 DE 102009057962
(43) Veröffentlichungstag der Anmeldung: 17.10.2012
(73) Patentinhaber: Karlsruher Institut für Technologie, 76131 Karlsruhe (DE)
(72) Erfinder: BRETTHAUER, Georg, 76139 Karlsruhe (DE); URANUES, Selman, 8042 Graz (AT)
(74) Vertreter: Müller-Boré & Partner Patentanwälte PartG mbB
(86) Internationale Anmeldenummer: PCT/EP2010/007536
(87) Internationale Veröffentlichungsnummer: WO 2011/069670

(56) Entgegenhaltungen:
- WO-A1-02/47557
- WO-A1-97/37002
- WO-A2-2008/122044
- US-A- 5 656 605
- US-A- 6 090 117
- US-A1- 2003 211 130
- US-A1- 2009 024 150
- US-B2- 6 716 225
- LACOUR S P ET AL: "Long Micro-Channel Electrode Arrays: A Novel Type of Regenerative Peripheral Nerve Interface", IEEE TRANSACTIONS ON NEURAL SYSTEMS AND REHABILITATIONENGINEERING, IEEE SERVICE CENTER, NEW YORK, NY, US, vol. 17, no. 5, 1 October 2009 (2009-10-01), pages 454-460, XP011348191, ISSN: 1534-4320, DOI: 10.1109/TNSRE.2009.2031241

## Beschreibung

Der Anmeldegegenstand bezieht sich auf eine Nervenprothese, eine Nervenprothese zur Behandlung von durchtrennten Nerven und ein Verfahren zur Herstellung einer Nervenprothese.

Im Allgemeinen umfaßt ein Nervengewebe Nervenzellen Dendriten, Axone und Neuroglia. Nervengewebe können Nervensysteme bilden, wobei der Begriff Nervensystem menschliche oder tierische Nervensysteme umfaßt.

Nervenzellen (auch Neuronen oder Nervenzellköper genannt) nehmen Reize bzw. Impulse auf oder lösen solche aus. Dendriten und Axone leiten Reize weiter. Markhaltige sowie marklose Axone sind von Neuroglia (Gliazellen) umgeben und übernehmen hauptsächlich stützende, ernährende und isolierende Funktionen.

Allgemein gesprochen besitzt eine Nervenzelle (Neuron) ein oder mehrere Dendriten und in der Regel ein Axon, die eine Verbindung zu anderen Nervenzellen darstellen, um mit ihnen zu kommunizieren. Dendriten dienen als Empfänger von Informationen. Hingegen ist ein Axon für die Übertragung von Informationen von einer Nervenzelle zu einer anderen zuständig. Zudem kann auch ein Nervenzellkörper selbst Informationen und somit Signale empfangen. Ein Axon leitet Informationen in Form von elektrischen Signalen zu Synapsen und damit an andere Nervenzellen weiter. Ein Axon kann je nach Typ der Nervenzelle von 1 µm bis 1 m und länger sein. Die Axone der Nervenzellen von Säugetieren weisen etwa eine Dicke von 0,5 bis 10 µm auf.

Die Signale zur Übertragung von Informationen von oder zu einer Nervenzelle sind elektrische Signale. In anderen Worten geschieht die Übertragung der elektrischen Signale durch Nervenfasern. Aufgrund einer Biomembran eines Axons liegt an der Außenwand des Axons eine andere elektrische Ladung an als in einem Innenbereich des Axons. Diese Ladungsdifferenz wird auch Polarisation oder Ruhepotential genannt.

Signalübertragungen bestehen unabhängig von der Klasse einer Nervenzelle immer aus Änderungen im elektrischen Potenzial über einer Plasmamembran einer Nervenzelle bzw. eines Neurons. Änderungen, welche auch Wellen elektrischer Erregung sind, werden auch als Aktionspotenziale bzw. Nervenimpulse bezeichnet.

Insbesondere können im Folgenden unter Nervenzellen sämtliche Klassen von Nervenzellen, nämlich beispielsweise unipolare Nervenzellen, bipolare Nervenzellen, pseudounipolare Nervenzellen sowie multipolare Nervenzellen, verstanden werden.

Im Folgenden ist unter dem Begriff Nerv eine Nervenzelle und insbesondere eine Nervenfaser zu verstehen. Insbesondere ist hierin unter dem Begriff Nerv auch ein Teil bzw. Teilbereich einer Nervenzelle zu verstehen. Weiterhin ist im Folgenden hierin unter dem Begriff Nervbereich ein Abschnitt, Teil oder Bereich einer Nervenzelle bzw. eines Nervs zu verstehen.

Werden die Nervenfasern für die Signalweiterleitung unterbrochen, können folglich keine Informationen von oder zu einer Sinneszelle übertragen werden. Die jeweilige Sinneszelle, z.B. eines Organs, eines Hautbereichs oder eines Muskels, wird dadurch in ihrer Funktion gestört. Beispielsweise können Gefühllosigkeiten auftreten und Empfindungen können nicht mehr bewußt wahrgenommen werden. Ebenso können Nervenzellen des Rückenmarks oder des Gehirns Informationen zur Steuerung von Muskeln und Drüsen nicht mehr übermitteln, so daß betroffene Muskeln und Drüsen untätig bleiben.

Beispielsweise werden bei einer Querschnittslähmung Nerven bzw. Nervenfasern, die durch das Rückenmark verlaufen, in der Signalweiterleitung unterbrochen. Aufgrund dessen fehlt bei einer Querschnittslähmung sowohl das Gefühl aus den abgeschnittenen Sinneszellen als auch die Arbeit und Flüssigkeitsproduktion von Muskeln und Drüsen des betroffenen Bereiches.

In anderen Worten kommt es bei Durchtrennungen sowie bei Verletzungen von Nerven zu einer teilweisen oder sogar vollständigen Unterbrechung von Nervensignalen. Als Folge von Störungen der Nervensignale wird eine Degeneration der betroffenen Nerven verursacht. Infolgedessen werden betroffene Lebewesen, wie z.B. Menschen oder Tiere in ihrer normalen Bewegungstätigkeit sowie weiteren Körperfunktionen stark beeinträchtigt. Insbesondere ist es problematisch, daß Nerven nach Durchtrennung sowie bei Verletzung in der Regel absterben, bevor ein Zusammenwachsen erfolgen konnte. Somit kann sich eine dauerhafte Unterbrechung von Nervensignalen einstellen, bevor durch ein Zusammenwachsen eine normale und unbeeinträchtigte Übertragung von Nervensignalen wiederhergestellt ist.

Druckschrift US 6,090,117 offenbart ein künstliches Röhrchen, welches aus zwei Ummantelungsschichten, einem inneren Röhrchen sowie einem Collagen-Körper besteht. Das künstliche Röhrchen mit den beiden Ummantelungsschichten bildet einen Hohlzylinder, wobei der Collagen-Körper in einem Hohlzylinder angeordnet ist. Die Ummantelungsschichten beinhalten Gelatine oder Collagen. Das künstliche Röhrchen besteht ebenfalls aus biologisch abbaubarem und im lebenden Organismus absorbierbaren Material. Der Collagen-Körper enthält mit einem Gel gefüllte Aussparungen.

Dokument US 5,656,605 offenbart eine Vorrichtung, um die Regeneration von beschädigten Nerven zu fördern, wobei die Vorrichtung ein polymerisches Führungsrohr mit einer darin enthaltenen therapeutischen Mischung umfasst. Weiterhin umfasst die Vorrichtung Führungsfasern, die sich entlang des Führungsröhrchens erstrecken.

WO 97/37902 offenbart eine Vorrichtung zur Förderung der Regeneration von Gewebe in einem Wundbereich. Die Vorrichtung umfasst ein Silikon-Führungsröhrchen und Führungsfasern sowie zusätzliche Hemmstoffe, um ein kohärentes Fibrin-Netzwerk auszubilden.

Die Druckschrift WO 2008/122044 A2 offenbart eine implantierbare medizinische Vorrichtung aufweisend ein elektrisch isolierendes Substrat, auf dessen Oberfläche eine Interface-Einheit angeordnet ist, welche eine Kommunikation mit einem biologischen Gewebe ermöglichen soll. Ferner ist ein polymerischer Mikrokanal-Elektrodenträger aus LACOUR S P et al: "Long Micro-Channel Electrode Arrays: A Novel Type of Regenerative Peripheral Nerve Interface" bekannt.

Es ist Aufgabe der vorliegenden Erfindung, bei geschädigten oder durchtrennten Nervenfasern eine Übertragung von Nervensignalen wiederherzustellen. Erfindungsgemäß wird diese Aufgabe durch die Merkmale der unabhängigen Ansprüche gelöst. Bevorzugte Ausführungsformen der Erfindung sind Gegenstand der abhängigen Ansprüche. Alle im Folgenden genannten Aspekte, Beispiele und Ausführungen, die nicht unter die unabhängigen Ansprüche fallen, sind nicht Teil der Erfindung.

### Nervenprothese gemäß einem Aspekt

Ein Aspekt der vorliegenden Erfindung betrifft eine Nervenprothese, umfassend:
eine Kopplungselementaufnahme, und
zumindest ein hohlförmig ausgebildetes Kopplungselement, welches einen ersten und einen zweiten Kopplungsabschnitt aufweist, wobei
das zumindest eine Kopplungselement durch die Kopplungselementaufnahme festgelegt ist, und wobei
das zumindest eine Kopplungselement (106, 106', 206, 306, 406) zumindest teilweise aus nicht-resorbierbarem Material besteht, und wobei das nicht-resobierbare Material ein Metall und/oder eine Metallegierung umfasst, und wobei der erste Kopplungsabschnitt an einem ersten Nervbereich anordenbar ist und der zweite Kopplungsabschnitt an einem zweiten Nervbereich anordenbar ist, so daß die beiden Nervbereiche mittels der Nervenprothese koppelbar sind.
Vorteilhafterweise wird mittels der Nervenprothese eine Nervenregeneration ermöglicht und eine Muskelatrophie, auch Muskelverkümmerung oder Gewebeschwund genannt, verhindert. Weiterhin vorteilhafterweise beschleunigt die Nervenprothese die körpereigenen Reparationsvorgänge und stellt die Signalübertragung über die verletzte Nervenregion wieder her.

Insbesondere ist unter einer Nervenprothese ein Zwischenstück zu verstehen, welches zwischen ein proximales und ein distales Ende eines Nervs bzw. einer Nervenfaser eingesetzt werden kann. Durch eine Nervenprothese kann eine Verbindung zweier voneinander getrennter Nervbereiche bzw. -enden eines Nervs hergestellt werden. Mit anderen Worten dient die Nervenprothese als Verbinder eines ersten Nervbereichs mit einem zweiten Nervbereich. Insbesondere sind unter einem ersten bzw. einem zweiten Nervbereich ein distales bzw. proximales Nervenende bzw. -bereich zu verstehen. Die beiden Nervbereiche können z.B. durch ein Durchtrennen eines Nervs entstehen. Die beiden Nervbereiche können sich ausgehend von einer Durchtrennungsstelle gegenüber liegen.

### Ausführungsformen der Nervenprothese

Die Nervenprothese umfaßt zumindest eine Kopplungselementaufnahme, welche einen Basis- bzw. Grundkörper bildet. Vorteilhafterweise ist der Basiskörper bzw. ist die Kopplungselementaufnahme in der Ausgestaltung, d.h. in seinen/ihren strukturellen Gestaltung an bestimmte Anwendungsbereiche in einem menschlichen oder tierischen Körper angepaßt. Entsprechend können sich Kopplungselementaufnahmen je nach Anwendungsbereich bzw. in der anzuwendenden Körperregion in ihrer geometrischen Ausgestaltung oder Dimensionierung unterscheiden. Beispielsweise kann eine Kopplungselementaufnahme eine im wesentlichen rundliche, ovale oder eine flache, längliche Ausgestaltung aufweisen. Beispielsweise kann die Kopplungselementaufnahme im Querschnitt z.B. im wesentlichen kreisförmig oder oval oder rechteckig sein. Dies gilt insbesondere für einen Querschnitt in einer zu einer Längsachse der Kopplungselementaufnahme senkrechten Schnittebene. Zudem können Nervenprothesen im wesentlichen symmetrisch genauso wie asymmetrisch ausgestaltet sein.

Insbesondere kann eine Kopplungselementaufnahme zumindest einen ersten und einen zweiten Kontaktbereich umfassen, wobei der erste und der zweite Kontaktbereich an einen ersten bzw. zweiten Nervbereich anordenbar sind. Vorzugsweise kontaktiert der erste Kontaktbereich einen ersten Nervbereich, z.B. einen proximalen Nervbereich. Vorzugsweise kontaktiert der zweite Kontaktbereich einen zweiten Nervbereich, z.B. einen distalen Nervbereich. Vorzugsweise können der erste bzw. zweite Kopplungsabschnitt eines Kopplungselements an einem jeweiligen Kontaktbereich der Kopplungselementaufnahme angeordnet sein.

Vorzugsweise umfaßt die Kopplungselementaufnahme zumindest ein Kopplungselement, welches einen ersten und einen zweiten Kopplungsabschnitt aufweist. Weiterhin vorzugsweise ist das zumindest eine Kopplungselement durch die Kopplungselementaufnahme festgelegt.

Unter dem Begriff festgelegt ist insbesondere zu verstehen, daß das Kopplungselement durch die Kopplungselementaufnahme gehalten bzw. fixiert wird. Insbesondere ist durch die Kopplungselementaufnahme eine Ausrichtung des Kopplungselements zur Kopplungselementaufnahme definierbar bzw. bestimmbar. Beispielsweise können das Kopplungselement und die Kopplungselementaufnahme eine chemische und/oder mechanische Bindung eingehen. Eine chemische Bindung kann z.B. durch Kleben erfolgen. Eine mechanische Bindung kann z.B. durch einen Reibschluß erfolgen.

Eine Ausrichtung eines Kopplungselements relativ zu einer Kopplungselementaufnahme wird vorzugsweise vor dem Einsetzen einer Nervenprothese zwischen einem distalen und einem proximalen Nervenende bestimmt. Vorzugsweise ist ein Kopplungselement derart an einer Kopplungselementaufnahme angeordnet bzw. durch eine Kopplungselementaufnahme festgelegt, daß ein erster Kopplungsabschnitt in dem ersten Kontaktbereich der Kopplungselementaufnahme und ein zweiter Kopplungsabschnitt in dem zweiten Kontaktbereich der Kopplungselementaufnahme angeordnet ist.

Der erste Kopplungsabschnitt sowie der zweite Kopplungsabschnitt des Kopplungselements sind jeweils an einem ersten bzw. einem zweiten Nervbereich anordenbar.

Insbesondere ist unter der Terminologie, daß ein Kopplungsabschnitt an einem Nervbereich anordenbar ist, auch zu verstehen, daß ein Kopplungsabschnitt eines Kopplungselements mit einem Nervbereich in Kontakt bringbar ist bzw. in Eingriff bringbar ist. Insbesondere ist ein Kopplungsabschnitt derart an einen Nervbereich anordenbar, so daß sich ein Kopplungsabschnitt und ein Nervbereich lediglich berühren bzw. tangieren.

In einer weiteren Ausgestaltung können ein Kopplungsabschnitt und ein Nervbereich derart aneinander angeordnet sein, daß ein Kopplungsabschnitt von einem Nervbereich umgeben bzw. umschlossen ist. In diesem Fall kann der Kopplungsabschnitt durch Einführen oder Einstechen in das Gewebe eines Nervbereichs angeordnet sein. Ebenfalls vorzugsweise kann ein Nervbereich von einem Kopplungsabschnitt umgeben bzw. umschlossen sein. Hierbei kann der Kopplungsabschnitt hülsen- oder kappenartig über einen Nervbereich gestülpt sein bzw. einen Nervbereich bedecken.

Weiterhin vorzugsweise können ein Kopplungselement und ein jeweiliger Nervbereich miteinander fixiert sein. Insbesondere kann eine Fixierung über ein Zusammenheften oder ein Zusammenkleben erfolgen. Weiterhin kann eine Fixierung durch ein Verklemmen von Gewebe des Nervbereichs mit dem Kopplungselement erzielt werden. Auch kann durch ein Einhaken des Kopplungselements in Gewebe des Nervbereichs eine Fixierung bzw. Halterung erzielt werden.

Somit ist vorzugsweise der erste Kopplungsabschnitt an einem ersten Nervbereich anordenbar und der zweite Kopplungsabschnitt an einem zweiten Nervbereich anordenbar, so daß die beiden Nervbereiche mittels der Nervenprothese koppelbar sind.

Weiterhin vorzugsweise kann die Nervenprothese mehr als ein Kopplungselement umfassen. Die Anzahl der Kopplungselemente kann hierbei abhängig von der zu behandelnden Körperregion oder des zu behandelnden Lebewesens sein. Insbesondere können 2 bis 6, vorzugsweise 3, Kopplungselemente für die Anwendung im menschlichen Körper eingesetzt werden. Möglich ist aber auch eine höhere Anzahl von Kopplungselementen beim Einsatz einer Nervenprothese im menschlichen Körper. Beispielsweise können 4, 5, 6, 7, 8, 9, 10, 15, 20, usw. Kopplungselemente vorhanden sein. Insbesondere bei Tieren kann in Abhängigkeit von der Tierart und der zu behandelnden Körperregion bzw. des zu behandelnden Nervs die Anzahl der zu verwendenden Kopplungselemente variieren.

Vorzugsweise kann eine Nervenprothese eine Kopplungselementaufnahme umfassen, welche eine Mehrzahl röhrenförmiger Leitelemente beinhaltet. Insbesondere können die Leitelemente als Mikroröhren ausgestaltet sein.

Zudem können sich die Leitelemente in einer Längsrichtung in der Kopplungselementaufnahme erstrecken, wobei die Längsrichtung eine Richtung im wesentlichen parallel zu dem zumindest einem Kopplungselement ist. Im Weiteren kann unter Längsrichtung eine Richtung verstanden werden, welche im wesentlichen senkrecht zu einem oder zu beiden der Kontaktbereiche der Kopplungselementaufnahme ist. Mit anderen Worten kann unter einer Längsrichtung eine Richtung parallel zu einer Kopplungsachse verstanden werden, welche im wesentlichen normal zu den Kontaktbereichen ausgerichtet ist. Vorzugsweise ist die Kopplungsachse im wesentlichen im Zentrum bzw. zentral in einer Nervenprothese angeordnet. Die Kopplungsachse kann beispielsweise einer Symmetrielinie einer Nervenprothese entsprechen.

Vorzugsweise sind die röhrenförmigen Leitelemente hohlzylindrisch ausgestaltet. Mit anderen Worten weist ein Leitelemente vorteilhafterweise zumindest einen Durchlaß auf.

Insbesondere kann unter einem röhrenförmigen Leitelement ein Leitelement verstanden werden, welches zumindest einen Durchgang, eine Durchführung, einen Kanal, einen Durchlaß bzw. eine Passiermöglichkeit von Fluiden aufweist. Mit anderen Worten ein röhrenförmiges Leitelement weist einen Hohlbereich auf.

Es ist zu verstehen, daß eine geometrische Ausgestaltung eines röhrenförmigen Leitelements vielfältig sein kann. Beispielsweise kann das röhrenförmige Leitelement einen ovalen oder mehreckigen Durchlaß aufweisen. Weiterhin kann ein röhrenförmiges Leitelement mehr als einen Durchlaß aufweisen, wobei die Durchlässe nicht die gleiche Dimensionierung aufweisen müssen. Vorzugsweise erstreckt sich ein Durchlaß bzw. ein Fluidkanal eines röhrenförmigen Leitelements von dem ersten Kontaktbereich zu dem zweiten Kontaktbereich der Kopplungselementaufnahme. Insbesondere kann die Dimensionierung der Durchlässe von der konkreten zu behandelnden Körperregion abhängen.

Weiterhin können die Dimensionierungen der Durchlässe einer Mehrzahl röhrenförmiger Leitelemente einer Kopplungselementaufnahme variieren. Daher können beispielsweise röhrenförmige Leitelemente einer Kopplungselementaufnahme mit einem größer dimensionierten Durchlaß bevorzugt im Rand- bzw. Außenbereich der Kopplungselementaufnahme angeordnet sein. In einer anderen Ausführung können röhrenförmige Leitelemente einer Kopplungselementaufnahme mit einem kleiner dimensionierten Durchlaß bevorzugt im Rand- bzw. Außenbereich der Kopplungselementaufnahme angeordnet sein. Ebenso können röhrenförmige Leitelemente mit größer sowie mit kleiner dimensioniertem Durchlaß in einem Zentralbereich und zugleich in einem Randbereich einer Kopplungselementaufnahme angeordnet sein.

Insbesondere kann ein röhrenförmiges Leitelement derart gestaltet sein, daß Gewebe eines Nervbereichs durch das röhrenförmiges Leitelement wachsen kann. Weiterhin vorzugsweise erlaubt ein Leitelement die Migration von Bestandteilen wie beispielsweise Blut, Wachstumshormonen, Zellen von dem ersten Nervbereich zu dem zweiten Nervbereich. Mit anderen Worten erlaubt ein röhrenförmiges Leitelement den Durchlaß von Fluiden.

Weiterhin vorzugsweise kann ein röhrenförmiges Leitelement ein elastisches bzw. nachgebendes Material umfassen, so daß ein Schädigen von an einem Nerv angrenzendem Gewebe bzw. Zellmaterial sowie dem Nerv selbst vermeidbar ist. Insbesondere kann/können ein oder mehrere der röhrenförmigen Leitelemente vollständig aus dem elastischen bzw. nachgebenden Material bestehen.

Weiterhin vorzugsweise umfaßt bzw. beinhaltet eine Kopplungselementaufnahme ein oder mehrere stabförmige Leitelemente. Stabförmige Leitelemente weisen insbesondere keinen Durchlaß bzw. Fluidkanal auf. Ebenso wie die röhrenförmigen Leitelemente können die stabförmigen Leitelemente nach einer bestimmten Strukturierung (Verteilung) in einer Kopplungselementaufnahme angeordnet sein. Vorzugsweise erstrecken sich die stabförmigen Leitelemente analog zu den röhrenförmigen Leitelementen in Längsrichtung einer Kopplungselementaufnahme.

Ebenfalls kann die äußere Geometrie eines röhren- bzw. stabförmigen Leitelements vielfältig ausgestaltet sein. Zum Beispiel kann ein röhren- sowie ein stabförmiges Leitelement die äußere Gestalt eines Quaders, eines Kegelstumpfes oder eines Prismas haben. Insbesondere kann durch die Geometrie eines röhren- bzw. stabförmigen Leitelements dessen mechanischen Eigenschaften hinsichtlich Biegungs-, Torsions-, Druck- sowie Zugbelastungen derart bestimmt werden, so daß diese Eigenschaften für den jeweiligen Einsatz bzw. für den jeweiligen Anwendungsfall optimiert sind. Insbesondere können eine Mehrzahl röhren- und/oder stabförmiger Leitelemente einer Kopplungselementaufnahme voneinander verschiedene geometrische Ausgestaltungen aufweisen.

Weiterhin vorzugsweise bildet ein Bündel bzw. eine Mehrzahl von röhren- und/oder stabförmigen Leitelementen die Kopplungselementaufnahme. Insbesondere können die röhren- und/oder stabförmigen Leitelemente im wesentlichen lose aneinander angeordnet sein und durch einen Form- und/oder Reib- und/oder Kraftschluß wie beispielsweise durch ein Halteband aneinander fixiert sein. Weiterhin können die röhren- und/oder stabförmigen Leitelemente im wesentlichen durch eine stoffschlüssige Verbindung wie beispielsweise Schmelzen oder Kleben aneinander fixiert sein.

Vorzugsweise können in einem röhrenförmigen Leitelement, welches im Vergleich zu anderen stab- und/oder röhrenförmigen Leitelementen der gleichen Kopplungselementaufnahme eine größere Dimensionierung, insbesondere einen größeren Durchmesser aufweist, stab- und/oder röhrenförmige Leitelemente mit einer relativ dazu kleineren Dimensionierung, insbesondere einem relativ dazu kleineren Durchmesser, in dem Durchlaß des größeren Leitelements anordenbar sein. In anderen Worten kann der Innendurchmesser des größeren Leitelements gleich oder größer als der Außendurchmesser des kleineren Leitelements sein.

Weiterhin bevorzugt ist zumindest eines der röhren- und/oder stabförmigen Leitelemente einer Kopplungselementaufnahme als stabilisierendes Element ausgebildet. Insbesondere weist ein stabilisierendes Element eine höhere Festigkeit auf als andere Leitelemente, so daß jedes stabilisierende Leitelement, verglichen zu einem röhren- und/oder stabförmigen Leitelement, das in diesem Zusammenhang auch als ein nicht stabilisierendes Leitelement bezeichnet werden kann, weniger leicht biegbar sein kann.

Beispielsweise kann ein röhren- und/oder stabförmiges Leitelement, welches als stabilisierendes Element ausgebildet ist, ein metallisches Material umfassen, während Leitelemente mit einer geringeren Festigkeit Gelatine beinhalten. Weiterhin vorzugsweise kann ein stabilisierendes Element als Material Gelatine umfassen, welche eine längere Molekülkette aufweist als andere röhren- und/oder stabförmigen Leitelemente mit einer geringeren Festigkeit. Es ist auch möglich, daß alle Leitelemente aus identischem Material gebildet sind, wobei die stabilisierenden Leitelemente jedoch kompakter gebildet sind, z.B. durch eine Ausbildung als solider bzw. massiver Körper, d.h. Hohlkörper frei.

Weiterhin bevorzugt ist das zumindest eine Kopplungselement in der Kopplungselementaufnahme angeordnet, wobei der erste und der zweite Kopplungsabschnitt über einen ersten bzw. zweiten Kontaktbereich der Kopplungselementaufnahme hinausragen. Vorzugsweise ist das zumindest eine Kopplungselement in oder neben einem Leitelement angeordnet.

Bevorzugt stehen die Kopplungsabschnitte um etwa 0,1 µm bis etwa 8 mm oder um etwa 5 mm bis 3 cm aus dem Kontaktbereich der Kopplungselementaufnahme hervor. Mit anderen Worten können die Kopplungsabschnitte eine Länge von etwa 1 µm oder länger aufweisen. Weiterhin vorzugsweise weisen die Kopplungsabschnitte eine Weite von etwa 0,1 µm bis 3 mm auf.

Weiterhin vorzugsweise weist jedes Kopplungselement zumindest zwei Kopplungsabschnitte auf. In anderen Worten sind die zumindest 2 Kopplungsabschnitte im wesentlichen gegenständig bzw. gegenüberliegend zueinander angeordnet. Die Enden der Kopplungsabschnitte sind entweder spitz oder stumpf. Besonders bevorzugt weist jedes Kopplungselement 3, 4, 5, 6, usw. Kopplungsabschnitte auf. In anderen Worten weist ein Kopplungselement mit mehr als 2 Kopplungsabschnitten bzw. mehreren Enden an einem Kopplungsabschnitt eine gabelförmige Verzweigung auf.

Insbesondere ist in diesem Zusammenhang unter dem Begriff spitz zu verstehen, daß ein Ende eines Kopplungsabschnitts eine Verjüngung oder eine abnehmende Abstufung zum Ende hin und/oder zumindest einen spitzen Winkel aufweist. Insbesondere können ein kegel- oder ein pyramidenförmiges sowie ein spritzen- bzw. injektionsnadelförmiges Ende als spitz betrachtet werden.

Hingegen ist in diesem Zusammenhang unter dem Begriff stumpf ein eher flaches, abgerundetes oder nur geringfügig verjüngtes Ende eines Kopplungsabschnitts zu verstehen. Insbesondere schließt die Terminologie "spitzes bzw. stumpfes Ende des Kopplungselements" die Ausgestaltung einer Körperkante und/oder eines Wandungsbereichs bzw. -fläche eines Endes eines Kopplungsabschnittes ein. Beispielsweise kann ein spitzes Ende scharfkantig sein und/oder scharfschneidende Eigenschaften aufweisen. Insbesondere ist eine Schnittebene, welche entlang eines spitzen bzw. stumpfen Endes verläuft, im wesentlichen in einem spitzen bzw. stumpfen Winkel zur Kopplungsachse einer Nervenprothese angeordnet.

Erfindungsgemäß ist das zumindest eine Kopplungselement hohl. Mit anderen Worten umfaßt ein Kopplungselement zumindest einen Fluidkanal, welcher einen Verbindungskanal zwischen den beiden Enden des Kopplungselements und somit zwischen dem ersten und dem zweiten Kopplungsabschnitt darstellt.

Vorzugsweise ist ein Kopplungselement röhrenförmig ausgestaltet. Bevorzugt ist ein Kopplungselement hohlzylindrisch bzw. als röhrenförmiges Element ausgestaltet. Weiterhin bevorzugt kann ein Kopplungsabschnitt ähnlich oder gleich einer Spritzen- bzw. Injektionsnadel, insbesondere ähnlich oder gleich einem Endbereich einer Spritzen- bzw. Injektionsnadel geformt sein.

Beispielsweise kann in einer Ausgestaltung eines Kopplungsabschnitts der Kopplungsabschnitt eine Weite aufweisen, so daß ein Axon, welches innerhalb eines Nervbereichs angeordnet ist, im wesentlichen innerhalb des Hohlzylinders angeordnet ist. Weiterhin bevorzugt weist ein Kopplungsabschnitt eine Weite auf, so daß eine Nervenfaser bzw. ein Axon zumindest teilweise innerhalb des Hohlzylinders des Kopplungsabschnitts angeordnet ist.

In einer weiteren Ausgestaltung kann zumindest ein Kopplungsabschnitt eine oder mehrere Ausnehmungen in einer Mantelfläche bzw. Wandung eines hohlzylindrischen Kopplungsabschnitts aufweisen. Eine solche Ausnehmung stellt einen Ein- bzw. Auslaß dar, welche zusätzlich oder alternativ ein Anordnen bzw. ein Einführen zumindest eines Teils eines Nervbereichs innerhalb des Hohlzylinders des Kopplungsabschnitts erlaubt.

Vorzugsweise können Kopplungselemente zumindest einen Kopplungsabschnitt aufweisen, welcher ein hohlzylindrisches Ende mit einem Durchlaß aufweist, und einen Kopplungsabschnitt, welcher ausschließlich in der Wandung des Kopplungsabschnitts zumindest einen Durchlaß aufweist.

Weiterhin kann eine Nervenprothese mehrere Kopplungselemente mit voneinander verschieden gestalteten Kopplungsabschnitten beinhalten.

Weiter bevorzugt weist das zumindest eine Kopplungselement zumindest eine Verzweigung auf, von welcher zumindest ein dritter Kopplungsabschnitte abzweigt.

Insbesondere bevorzugt ragen zwei Kopplungsabschnitte bei einem Kopplungselement, welches einen ersten bis dritten Kopplungsabschnitt beinhaltet, über einen ersten Kontaktbereich hinaus und ein dritter Kopplungsabschnitt über einen zweiten Kontaktbereich hinaus. Mit anderen Worten kann ein Kopplungselement eine gabelartige Ausformung aufweisen. Weiterhin kann ein Kopplungselement mehr als eine Verzweigung beinhalten, so daß beispielsweise über jeden Kontaktbereich einer Kopplungselementaufnahme mindestens zwei Kopplungsabschnitte hinausragen, so daß ein Kopplungselement mindestens vier Kopplungsabschnitte aufweist.

Weiterhin vorzugsweise kann eine Kopplungselementaufnahme mehr als zwei Kontaktbereiche aufweisen. Beispielsweise kann eine Kopplungselementaufnahme drei Kontaktbereiche aufweisen, wobei jeweils ein Kopplungsabschnitt eines Kopplungselements über jeden Kontaktbereich hinausragt.

Vorteilhafterweise ermöglichen Kopplungselemente ein gezieltes Zusammenwachsen von Nervbereichen und ermöglichen den ersten Kontakt zwischen den getrennten Nervenbahnen bzw. -bereichen.

Weiters bevorzugt umfaßt eine Nervenprothese einen Mantel. In einer bevorzugten Ausführung sind die Kopplungselementaufnahme und zumindest teilweise das zumindest eine Kopplungselement innerhalb des Mantels angeordnet.

In einer Ausgestaltung kann ein Kopplungsabschnitt weiter über einen Kontaktbereich bzw. eine Kopplungselementaufnahme hinausragen als ein Bereich eines Mantels über die Kopplungselementaufnahme hinausragt und umgekehrt.

Vorzugsweise stabilisiert der Mantel die Kopplungselementaufnahme gegen destruktive mechanische Einwirkungen. Im Anordnungszustand kann der Mantel insbesondere einen angeordneten Nerv, insbesondere die in den Kopplungsabschnitten angeordneten Nervbereiche schützen. Weiterhin kann auch die Verbindung zwischen den Nervbereichen und Kopplungsabschnitten geschützt und stabilisiert werden. Zudem kann der Mantel auch die Kopplungselemente vor Krafteinwirkungen schützen. Weiterhin kann der Mantel ein Material beinhalten, welches die Kopplungselementaufnahme und das zumindest eine Kopplungselement gegen chemische Einwirkungen stabilisiert.

Bevorzugt ist der Mantel im wesentlichen ein Hohlzylinder. In anderen Worten können die Kontaktbereiche und die Kopplungsabschnitte von dem Mantel ausgespart bzw. von einer Umhüllung durch den Mantel ausgenommen sein.

Insbesondere ragt der Mantel vorzugsweise von etwa 0,1 µm bis etwa 8 mm über die Kopplungselementaufnahme hinaus. Weiterhin vorzugsweise ragt der Mantel von etwa 0,2 µm bis etwa 20 mm, besonders bevorzugt zwischen etwa 2 mm bis etwa 15 mm über die Kopplungselementaufnahme hinaus. Anders ausgedrückt erstreckt sich der Mantel in Längsrichtung von der Kopplungselementaufnahme jeweils in Richtung der Enden der Kopplungselemente.

Weiterhin vorzugsweise umfangen die Bereiche des Mantels den ersten und den zweiten Kontaktbereich der Kopplungselementaufnahme. Vorzugsweise legen diese Bereiche des Mantels jeweils einen ersten bzw. einen zweiten Adaptionsbereich fest. Bevorzugt weist der jeweilige Adaptionsbereich eine Weite von etwa 0,5 µm bis etwa 2,8 mm oder von etwa 2,5 mm bis etwa 10 mm oder von etwa 2,3 mm bis etwa 15 mm auf.

Bevorzugt ist die Weite des jeweiligen Adaptionsbereichs gleich oder größer als die Weite des jeweiligen Nervbereichs.

Vorteilhafterweise dient der Mantel, welcher auch als Umhüllung definierbar ist, insbesondere einem stabilen Zusammenfügen getrennter Nervenenden bzw. - bereiche und ermöglicht die leichte Handhabbarkeit bei einem operativen Eingriff bzw. während eines Operationsvorgangs zum Einsatz der Nervenprothese.

In einer weiteren Ausgestaltung ist die Nervenprothese als integrales Element ausgestaltet. Hierbei umfaßt die Nervenprothese einen oder mehrere Kanäle. In den Kanälen können zumindest teilweise Kopplungselemente angeordnet sein. Die Kanäle können in ihrer Ausgestaltung sowie Funktionalität ähnlich zu den röhrenförmigen Leitelementen sein. Entsprechend ist es beispielsweise möglich, daß die Kanäle eine Migration von Körperstoffen wie Hormonen, Blutkörperchen etc. erlauben.

Vorzugsweise beinhaltet eine Nervenprothese drei oder mehr Kopplungselemente. Die drei oder mehr Kopplungselemente können durch entsprechende Kanäle festgelegt sein.

Bevorzugt kann auch zumindest ein stabförmiges Leitelement in einem Kanal angeordnet sein. Weiterhin kann ein stabilisierendes Element in einem Kanal angeordnet sein.

Weiterhin vorzugsweise beinhalten der Mantel und die Kopplungselementaufnahme resorbierendes Material. Hierdurch kann beispielsweise nach einem durchschnittlich zu erwartenden oder einem vordefinierten bzw. festgelegten Heilungszeitraum sichergestellt werden, daß die Nervenprothese nach einer Absorptionszeit im wesentlichen absorbiert ist. Vorteilhafterweise kann durch eine Absorption von Bestandteilen einer Nervenprothese sichergestellt werden, daß sich Körpergewebe hindernisfrei bzw. wieder vollständig in einer behandelten Körperregion ausbreiten kann. Somit können langfristig nach einer Behandlung von durchtrennten Nerven die ursprünglichen Umgebungsbedingungen des abgeheilten Nervenmaterials sowie des dem Nerv umgebenden Gewebes im wesentlichen wieder hergestellt werden. Der Begriff resorbiert umfaßt somit insbesondere, daß das resorbierte Material aufgelöst ist. Somit können der Mantel und die Kopplungselementaufnahme nach der Absorption im wesentlichen vollständig aufgelöst, d.h. vom Körper abgebaut sein.

Vorteilhafterweise umfaßt die Nervenprothese resorbierende Materialien, welche eine voneinander unterschiedliche Absorptionszeit aufweisen. Insbesondere sind durch unterschiedliche Absorptionszeiten verschiedener Bereiche bzw. Elemente der Nervenprothese die Heilungsbedingungen eines durchtrennten Nervs über die Dauer des Heilungsprozesses steuerbar.

Beispielsweise kann es vorteilhaft sein, daß zumindest eine Mehrzahl der Leitelemente der Kopplungselementaufnahme einen kürzeren Absorptionszeitraum aufweist als der Mantel. Somit könnten die Leitelemente während eines Heilungsvorganges bereits teilweise absorbiert sein, so daß der Nerv und/oder das den Nerv umgebende Gewebe nahezu ungehindert wachsen bzw. sich regenerieren kann, während der Mantel noch nicht oder nur anfänglich absorbiert ist. Somit kann der Mantel noch eine ausreichende Stabilität gegen äußere Einflüsse aufweisen, welche das Nervenwachstum stören bzw. den Nerv schädigen könnten.

Weiterhin ist zumindest eines der Leitelemente als stabilisierendes Element ausgebildet, welches eine höhere Absorptionsbeständigkeit aufweist als andere Leitelemente.

Vorzugsweise ist resorbierendes Material Gelatine und/oder Polyglycolsäure.

Erfindungsgemäß besteht das zumindest eine Kopplungselement aus nicht-resorbierbarem Material. Erfindungsgemäß ist das nicht-resorbierbare Material ein Metall und/oder eine Metalllegierung. Insbesondere
kann Titan als das nicht-resorbierbare Material verwendet werden. Weiterhin bevorzugt werden Metalle bzw. Metalllegierungen verwendet, welche anerge und/oder antiallergene Eigenschaften haben. Insbesondere können chirurgische Stähle bzw. medizinisch verwendete Materialien bzw. Metalle verwendet werden.

Beinhaltet ein Kopplungselement sowohl resorbierbares als auch nichtresorbierbares Material, ist das resorbierbare Material vorzugsweise Gelatine.

Vorteilhafterweise ist die Absorptionszeit des resorbierenden Materials durch die Länge der Molekülkette festlegbar.

Vorzugsweise sind die Kopplungselementaufnahme und der Mantel aus resorbierenden Material, wobei die Absorptionszeit des Mantels gleich oder höher als die Absorptionszeit der Kopplungselementaufnahme ist.

Weiterhin bevorzugt ist die Nervenprothese nach einem Absorptionszeitraum von 9 Monaten im wesentlichen absorbiert. Je nach zu behandelnder Körperregion kann die Absorptionszeit so festgelegt werden, daß die Nervenprothese im wesentlichen nach 3 Monaten, nach 6 Monaten oder erst nach einem Zeitraum zwischen 12 und 24 Monaten resorbiert ist.

Insbesondere vorzugsweise ist die Nervenprothese im wesentlichen bioresorbierbar. Weiterhin vorzugsweise ist die Nervenprothese im wesentlichen biokompatibel.

Die Nervenprothese kann einen oder mehrere Stimulatoren umfassen, obwohl die Funktionsfähigkeit der vorangegangen beschriebenen Nervenprothese auch ohne Stimulator gegeben ist. In anderen Worten ist die Nervenprothese im wesentlichen frei von Stimulatoren. Insbesondere ist die Nervenprothese vollständig frei von Stimulatoren.

Bevorzugt kann die Nervenprothese zumindest einen biochemischen Stimulator umfassen. Der biochemische Stimulator kann ausgelegt sein, ein Koppeln der beiden Nervbereiche zu stimulieren. Vorzugsweise ist der zumindest eine Stimulator in einem oder mehreren der Leitelemente und/oder in einem oder mehreren der Kanäle angeordnet. Zusätzlich oder alternativ kann einer oder können mehrere biochemischen Stimulatoren in dem zumindest einem Kopplungselement angeordnet sein. Insbesondere sind unter dem Begriff Stimulator Aktivatoren zu verstehen, welche die Bildung von ungünstigen Heilungsbedingungen verhindern und/oder die Heilungsbedingungen verbessern.
Vorzugsweise werden die Stimulatoren zumindest zu Beginn des Absorptionszeitraums über die Kontaktbereiche der Kopplungselementaufnahme abgegeben.

Weiterhin vorzugsweise sind mechanische und/oder elektrische Stimulatoren an der Nervenprothese anordenbar.

Nervenstimulatoren sind Geräte, die über eine Dipolarisation an Nerven einen elektrischen Strom hervorrufen. Die Stromstärke und die Stromfrequenz (Frequenz) können je nach Bedarf unterschiedlich sein. Die Stimulationsbreite (Dauer der einzelnen Stimulationsimpulse) kann ebenfalls unterschiedlich lang sein. Auch können die zu stimulierenden Nervenfasern selektiv sensorische oder motorische Fasern sein. Auch Reizstromgeräte können zu diesem Zweck verwendet werden.

In anderen Worten, können elektrische Stimulatoren (Nervenstimulatoren) Geräte, Bauteile und insbesondere mikroelektronische und/oder elektromechanische Bauelemente sein, die über eine Dipolarisation an Nerven einen elektrischen Strom hervorrufen. Die Stromstärke und die Frequenz der elektrischen Stimulatoren können insbesondere je nach konkreter Anwendung bzw. zu behandelnder Körperregion und Lebewesen (Mensch, bestimmte Tierart) unterschiedlich sein. Die Stimulationsbreite der Geräte, unter der die Dauer der einzelnen Stimulationsimpulse zu verstehen ist, kann ebenfalls variabel einstellbar sein. Bei der Anwendung eines elektrischen Stimulators in einem zu behandelnden Körpers oder Nervs kann sich die Stimulation aus verschiedenen Stimulationsbreiten, wie beispielsweise alternierende Stimualtionsbreiten, zusammensetzen. Weiterhin können die zu stimulierenden Nervenfasern selektiv sensorische oder motorische Fasern sein. Ferner können zur Stimulation von Nervenfasern Reizstromgeräte eingesetzt werden.

Weiterhin vorzugsweise können je nach Anwendungsfall spezielle Stimulatoren angewendet werden. Bevorzugt werden universell einsetzbare Stimulatoren verwandt.

Beispielsweise können mechanische und/oder elektrische Stimulatoren an dem Mantel der Nervenprothese angeordnet sein. Zudem können mechanische und/oder elektrische Stimulatoren über Kopplungselemente, insbesondere Kopplungsabschnitte an einer Nervenprothese angeordnet sein.

Weiterhin bevorzugt ist an einer Nervenprothese zumindest ein chirurgischer Faden angeordnet. Der zumindest eine chirurgische Faden erlaubt bzw. ermöglicht ein Koppeln zumindest eines Nervbereichs an die Nervenprothese.

Vorzugsweise ist ein chirurgischer Faden in der Kopplungselementaufnahme angeordnet. Weiterhin vorzugsweise ist ein chirurgischer Faden durch die Kopplungselementaufnahme durchführbar.

Bevorzugt ist ein chirurgischer Faden in einem röhrenförmigen Leitelement angeordnet. Beispielsweise ist ein chirurgischer Faden in ein röhrenförmiges Leitelement eingeführt, wobei zumindest ein Fadenende aus zumindest einem der Kontaktbereiche hinausragt.

Weiterhin bevorzugt ist ein chirurgischer Faden an einer Nervenprothese fixiert oder befestigt.

### Nervenprothese zur Verwendung gemäß einem Aspekt

Ein Aspekt der Erfindung umfaßt eine Nervenprothese zur Verwendung in der Behandlung von durchtrennten Nerven.

Bevorzugt können ein oder mehrere Elemente bzw. Merkmale der Nervenprothese zur Verwendung in der Behandlung von durchtrennten Nerven ein oder mehrere Weiterbildungen bzw. Ausgestaltungen der voran beschriebenen Nervenprothese umfassen.

### Verfahren gemäß einem Aspekt

Ein Aspekt der Erfindung umfaßt ein Verfahren zum Herstellen einer Nervenprothese, welches die Schritte umfaßt:
- Bereitstellen einer Mehrzahl Leitelemente; und
- Anordnen zumindest eines hohlförmig ausgebildeten Kopplungselements an die Mehrzahl Leitelemente; wobei die Mehrzahl Leitelemente eine Kopplungselementaufnahme bildet, und wobei das Kopplungselement mittels der Kopplungselementaufnahme festgelegt wird.

Ein oder mehrere Kopplungselemente können auch jeweils in einem Leitelement angeordnet sein.

### Ausführungsvarianten des Verfahrens

Vorzugsweise, wird das zumindest eine Kopplungselement zumindest teilweise durch Pressen, Schmelzen oder Kleben mit der Kopplungselementaufnahme fixiert.

Bevorzugt umfaßt das Verfahren weiterhin den weiteren Schritt des Anordnens eines Mantels.

Vorzugsweise wird der Mantel durch Pressen, Schmelzen oder Kleben mit der Kopplungselementaufnahme zumindest teilweise fixiert.

Bevorzugt werden die Kopplungselementaufnahme, das zumindest eine Kopplungselement und der Mantel zumindest teilweise durch Pressen, Schmelzen oder Kleben aneinander fixiert.

Bevorzugt kann das Verfahren zum Herstellen einer Nervenprothese ein oder mehrere Aspekte hinsichtlich Nutzung bzw. Ausgestaltung der Nervenprothese umfassen.

Vorteilhafterweise kann die in dieser Anmeldung beschrieben Nervenprothese ohne weitere Hilfsmittel bzw. Zusatzeinrichtungen zum Überbrücken von Nervensignalen in einem zerstörten Nervengewebe bzw. geschädigten Nerv eingesetzt werden. Somit ist die Nervenprothese geeignet, um sehr einfach und schnell eine gestörte Nerventätigkeit wiederherzustellen. Insbesondere ist beim Einsatz einer solchen Nervenprothese in geschädigtes Gewebe nur ein geringer Zeitaufwand erforderlich. Zudem sind keine Spezialapparaturen oder ein spezielles Operationsumfeld erforderlich, somit ist ein breiter Anwendungsbereich der Nervenprothese möglich.

Zudem ist eine solche Nervenprothese einfach und kosteneffizient herstellbar und einsetzbar. Dadurch können entstehende Kosten hinsichtlich Behandlung und Pflege von Personen mit geschädigten Nerven reduziert werden bzw. eine Heilung einer Person bei geringen Kosten erzielt werden.

Insbesondere kann durch einen Einsatz der Nervenprothese in geschädigtes Gewebe die Weiterleitung der Nervenimpulse, die Nervenregeneration aber auch Muskelkontraktionen erreicht werden.

Vorteilhafterweise können durch den Einsatz einer Nervenprothese traumatische Nervenläsionen und nicht degenerative Folgezustände, wie bei Patienten mit einem Schlaganfall, einer gezielten Behandlung zugeführt werden. In weiterer Folge ist neben peripheren Nervenläsionen auch die Behandlung von Rückenmarkläsionen möglich.

Besonders vorteilhaft ist, daß die Nervenprothese resorbierbar, leicht handhabbar, Gewebe schonend bzw. geschmeidig und industriell herstellbar ist.

Weiterhin vorteilhafterweise kann die Nervenprothese in unterschiedlichen Dimensionen produziert werden, um eine möglichst optimale Anpassung am Gebrauchsort zu erzielen.

Insbesondere vorteilhaft ist, daß die Nervenprothese als Fertigprodukt ausgestaltet sein kann, welches in einfacher Weise ohne weitere Maßnahme in jedem Operationssaal gelagert und im Bedarfsfall sofort eingesetzt werden kann. Hierdurch kann eine der wichtigsten Voraussetzungen für eine Restitutio ad integrum, eine möglichst rasche Überbrückung des defekten Areals am Nerv erfüllt werden. Denn kurz nach dem Durchtrennen eines Nervs sind die traumabedingten Gewebereaktionen am Nerven (wie Ödem, Hämatom, Entzündung) nur gering vorhanden und an einem so genannten Erfolgsorgan bzw. Sinneszelle (wie beispielsweise der Muskulatur) können die atrophischen Reaktionen verhindert werden. Eine verspätet einsetzende Nervenfunktion würde hingegen einen langwierigen Vorgang des Muskelaufbaus im Versorgungsgebiet dieses Nervs zur Folge haben.

Weiterhin vorteilhaft ist, daß die Nervenprothese vom Körper nach Abschluß der Regeneration der jeweiligen Nerven über den Absorptionsweg aufgelöst wird. Es verbleiben beispielsweise nur mehr ein oder mehrere Kopplungselemente, welche als bestehende Elektroden im Nervengewebe dienen. Diese Elektroden (wie beispielsweise Kopplungselemente aus Titan) sind anerge und für die Nervenfunktion nicht störende Elemente. Darüber hinaus ist vorteilhaft, daß solche Kopplungselemente zudem zu einer erhöhten Gewebestabilität in einer ehemaligen Verletzungszone bzw. einer behandeltem Körperregion führen, wodurch ein besserer Schutz gegen äußere Einwirkungen (wie dies z.B. bei gleichzeitig vorliegender Knochenverletzung möglich ist) gewährleistet.

### Kurze Beschreibung der Figuren

Die Erfindung wird nachstehend anhand von Ausführungsbeispielen unter Bezugnahme auf die beigefügten Zeichnungen beispielhaft beschrieben, wobei der allgemeine Erfindungsgedanke nicht auf die Ausführungsbeispiele beschränkt werden soll. Insbesondere können einzelne Merkmale der vorangehend und/oder nachfolgend beschrieben Aspekte und/oder Ausführungsformen losgelöst von dem jeweiligen Aspekt bzw. der jeweiligen Ausführungsform zu weiteren Ausführungsformen kombiniert werden.

Es zeigt:
- Fig. 1:: eine perspektivische Darstellung einer Nervenprothese,
- Fig. 2:: eine schematische Darstellung eines Abschnittes einer Nervenprothese im Schnitt entlang einer Querrichtung,
- Fig. 3:: eine weitere schematische Darstellung eines Abschnittes einer Nervenprothese im Schnitt entlang einer Querrichtung,
- Fig. 4:: eine schematische Darstellung einer Nervenprothese im Schnitt entlang einer Längsrichtung,
- Fig. 5:: eine perspektivische Darstellung einer weiteren Ausgestaltung einer Nervenprothese,
- Fig. 6:: eine schematische Darstellung einer Ausgestaltung einer Kopplungselementaufnahme in der Draufsicht und in der Seitenansicht.

### Detaillierte Beschreibung der Figuren

Insbesondere können im Folgenden unter Nervenzellen sämtliche Klassen von Nervenzellen, nämlich beispielsweise unipolare Nervenzellen, bipolare Nervenzellen, pseudounipolare Nervenzellen sowie multipolare Nervenzellen, verstanden werden.

Im Folgenden ist unter dem Begriff Nerv eine Nervenzelle und insbesondere eine Nervenfaser zu verstehen. Insbesondere ist hierin unter dem Begriff Nerv auch ein Teil bzw. Teilbereich einer Nervenzelle zu verstehen. Weiterhin ist im Folgenden hierin unter dem Begriff Nervbereich ein Abschnitt, Teil oder Bereich einer Nervenzelle bzw. eines Nervs zu verstehen.

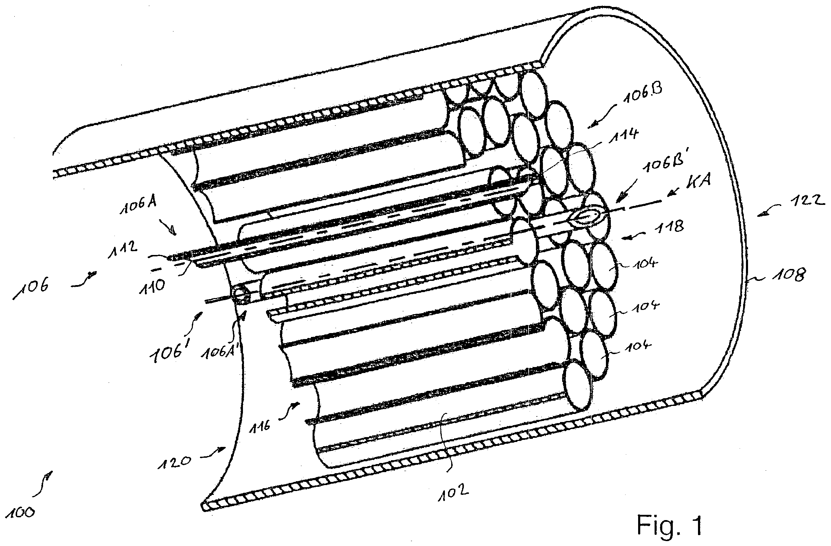

**Figur 1** zeigt eine perspektivische Darstellung einer Nervenprothese 100 mit einer Kopplungselementaufnahme 102, welche eine Mehrzahl Leitelemente 104, zwei Kopplungselemente 106, 106' und einen Mantel 108 umfaßt.

Jedes der zwei Kopplungselemente 106, 106' weist jeweils einen ersten Kopplungsabschnitt 106A, 106A' und einen zweiten Kopplungsabschnitt 106B, 106B' auf, wobei die beiden Kopplungselemente durch die Kopplungselementaufnahme 102 festgelegt sind. Die beiden Kopplungselemente 106, 106' sind jeweils an zwei Nervbereiche (nicht gezeigt) anordenbar.

Beispielsweise können die jeweils ersten Kopplungsabschnitte 106A, 106A' an einem ersten Nervbereich angeordnet sein und die jeweils zweiten Kopplungsabschnitte 106B, 106B' an einem zweiten Nervbereich angeordnet sein, wenn die Nervenprothese 100 zu einer Kopplung von voneinander getrennten Nervbereichen in einen zu behandelnden menschlichen Körper eingesetzt wurde. Zum Beispiel kann nach einem Einsetzen der Nervenprothese 100 ein erster Kopplungsabschnitt 106A mit einem ersten Nervbereich, welcher einem distalen Nervenende entspricht, gekoppelt sein, während ein zweiter Kopplungsabschnitt 106B mit einem zweiten Nervbereich, welcher einem proximalen Nervenende entspricht, gekoppelt ist.

Wie aus der Figur 1 ersichtlich ist, sind die Leitelemente 104 im wesentlichen röhrenförmig, daher können die Leitelemente 104 auch als röhrenförmige Leitelemente bezeichnet werden. Weiterhin sind die Leitelemente 104 im wesentlichen parallel zueinander angeordnet und im wesentlichen parallel entlang einer Längsrichtung der Nervenprothese 100 ausgerichtet.

Die Kopplungselementaufnahme 102 ist im wesentlichen durch die Leitelemente 104 gebildet. Die Leitelemente können in etwa eine gleiche oder ähnliche Dimensionierung in die Längsrichtung bzw. Kopplungsrichtung KA aufweisen. Die Kopplungsrichtung KA und die Längsrichtung sind vorzugsweise parallel. Ebenfalls kann, wie dargestellt, eine Dimensionierung quer zur Kopplungsrichtung KA gleich oder ähnlich sein. In anderen Worten kann der Durchmesser der Leitelemente ähnlich sein.

In einer modifizierten Ausführungsform der Nervenprothese 100 sind die Leitelemente 104 unterschiedlich von einander in eine Quer- und/oder Längsrichtung dimensioniert. Weiterhin können die Leitelemente eine von einer hohlzylindrischen Ausgestaltung abweichende Geometrie (wie Kegelstumpf oder Prisma) aufweisen. Insbesondere können durch die Dimensionierung und Geometrie der Leitelemente deren mechanischen Eigenschaften gesteuert werden, so daß die einzelnen Leitelemente und insbesondere die eine Mehrzahl Leitelemente umfassende Kopplungselementaufnahme im Hinblick auf einen konkreten Anwendungsfall bzw. auf eine zu behandelnde Körperregion abgestimmte mechanische Eigenschaften aufweisen.

In dieser Ausführungsform einer Nervenprothese entspricht die Längsrichtung einer Symmetrielinie der Nervenprothese sowie einer Kopplungsachse KA.

Weiterhin umfaßt die Nervenprothese 100 zwei Kontaktbereiche 116, 118, welche sich vorzugsweise im wesentlichen senkrecht zu der Kopplungsachse KA erstrecken. In anderen Worten werden die Kontaktbereiche 116, 118 durch die jeweiligen Enden der Leitelemente 104 gebildet.

Die Kontaktbereiche 116, 118 können als Ebenen ausgebildet sein, wie dies in Figur 1 gezeigt ist. In einer anderen Ausführungsform können die Kontaktbereiche unebene Flächen sein, welche Erhebungen bzw. Vertiefungen aufweisen. Insbesondere ist in diesem Zusammenhang der Begriff Erhebung bzw. Vertiefung derart zu verstehen, daß ein oder mehrere Leitelemente im Vergleich zu anderen Leitelementen in der Längsrichtung über andere Leitelemente hinausragen bzw. zurückversetzt sind.

Werden Nervbereiche an die Nervenprothese 100 gekoppelt, erfolgt dies im wesentlichen in Längsrichtung bzw. entlang der Kopplungsachse KA. Vorzugsweise können angekoppelte Nervbereiche den jeweiligen Kontaktbereich berühren bzw. tangieren.

Ebenfalls sind in der dargestellten Ausführungsform der Nervenprothese 100 die beiden Kopplungselemente 106, 106' parallel zu der Kopplungsachse KA angeordnet.

In einer modifizierten Ausführungsform der gezeigten Nervenprothese 100 ist es jedoch ebenfalls möglich, daß die Kopplungselemente 106, 106' nicht parallel, sondern in einem festgelegten bzw. bestimmten Winkel zu der Kopplungsachse KA angeordnet sind. Genauso können die Leitelemente 104 oder zumindest einige der Leitelemente 104 einen Winkel mit der Kopplungsachse KA einschließen.

Wie in der Figur 1 gezeigt ist, können die Kopplungselemente 106, 106' im wesentlichen hohlzylindrisch ausgebildet sein, so daß die Kopplungselemente einen Durchlaß bzw. einen Fluidkanal aufweisen. Beispielsweise umfaßt das Kopplungselement 106 einen Durchlaß 110, welcher sich von einem ersten Ende 112 des Kopplungselements 106 zu einem zweiten Ende 114 des Kopplungselements 106 erstreckt.

Gemäß der Figur 1 können die Enden 112, 114 eines Kopplungselements bzw. eines Kopplungsabschnitts 106A, 106B spitz ausgeformt sein. Dies bedeutet insbesondere, daß eine Schnittebene, welche beispielsweise entlang eines Endes 112 verläuft, die Kopplungsachse KA im wesentlichen in einem spitzen Winkel schneidet.

Gemäß einer weiteren Ausführungsform kann zumindest ein Ende eines Kopplungselements eine Schnittebene aufweisen, die im wesentlichen normal zu der Kopplungsachse KA verläuft.

Insbesondere ragen die Kopplungsabschnitte 106A-B', wie in Figur 1 gezeigt, der Kopplungselementaufnahme 102 heraus.

Weiterhin ist es ebenfalls möglich, daß ein Kopplungsabschnitt eine lokale Erhebung bzw. ein lokales Hervorstehen darstellt.

Insbesondere können die Kopplungsabschnitte je nach Dimensionierung eines Kopplungsabschnitts und/oder einer Nervenprothese um etwa 0,1 µm bis 5 µm, etwa 3 µm bis etwa 30 µm, etwa 25 µm bis etwa 1 mm, etwa 1 mm bis etwa 8 mm und/oder um etwa 5 mm bis etwa 3 cm aus dem Kontaktbereich der Kopplungselementaufnahme hervorstehen.

Die Nervenprothese 100 umfaßt weiterhin einen Mantel 108, welcher die Kopplungselementaufnahme 102 im wesentlichen umfängt bzw. umschließt. In anderen Worten sind die Kopplungselementaufnahme 102 und zumindest teilweise die Kopplungselemente 106, 106' innerhalb des Mantels angeordnet.

Der in Figur 1 dargestellte Mantel 108 ist im wesentlichen ein Hohlzylinder. Je nach Ausgestaltung einer Nervenprothese 100 kann der Mantel 108 um etwa 0,1 µm bis etwa 50 µm, etwa 30 µm bis etwa 3 mm und/oder 2 mm bis 8 mm oder mehr über die Kontaktbereiche 116, 118 der Kopplungselementaufnahme 102 hinausragen.

Weiterhin kann ein Mantel derart ausgestaltet sein, daß er nur die Kopplungselementaufnahme oder zumindest einen der Kopplungsabschnitte umfängt.

Wie in Figur 1 dargestellt, können die Bereiche des Mantels 108, welche über die Kopplungselementaufnahme 102 hinausragen, den ersten und den zweiten Kontaktbereich 116, 118 umfangen und jeweils einen ersten Adaptionsbereich 120 bzw. einen zweiten Adaptionsbereich 122 festlegen.

Der jeweilige Adaptionsbereich 120, 122 kann beispielsweise eine Weite von etwa 0,2 µm bis etwa 20 mm, bevorzugt von etwa 0,5 µm bis etwa 2,8 mm oder von etwa 2,5 mm bis etwa 10 mm aufweisen. Weiterhin kann der jeweilige Adaptionsbereich 120, 122 beispielsweise eine Weite von etwa 2,3 mm bis etwa 15 mm aufweisen.

Insbesondere kann ein Adaptionsbereich einen Nervbereich aufnehmen. Insbesondere kann ein Nervbereich durch einen Adaptionsbereich gestützt bzw. fixiert bzw. gehalten sein. Weiterhin kann durch einen Adaptionsbereich ein Nervbereich von äußeren Einflüssen im wesentlichen geschützt sein.

Die Leitelemente 104, welche die Kopplungselementaufnahme 102 umfaßt, sind durch den Mantel 108 festgelegt bzw. fixiert. Ebenfalls sind die Kopplungselemente 106, 106' durch den Mantel 108 festgelegt. Beispielsweise können die Leitelemente 104 und die Kopplungselemente 106, 106' durch kraftschlüssige Verbindung mit dem Mantel 108 in ihrer Anordnung festgelegt sein.

In einer modifizierten Ausgestaltung können die Leitelemente 104 miteinander formschlüssig oder stoffschlüssig verbunden sein. Der Mantel 108 kann ebenfalls form- oder stoffschlüssig mit der Kopplungselementaufnahme 102 verbunden sein. Alternativ ist der Mantel 108 kraftschlüssig mit der Kopplungselementaufnahme 102 verbunden, während die Leitelemente 104 miteinander stoffschlüssig verbunden sind.

In zumindest einem der Leitelemente 104 und/oder in den Kopplungselementen 106, 106' können Stimulatoren (nicht gezeigt) angeordnet sein, welche eine Wundheilung bzw. eine Nervenwachstum fördern bzw. begünstigen.

Vorzugsweise kann ein Stoffschluß über Gelatine als Klebemittel erfolgen.

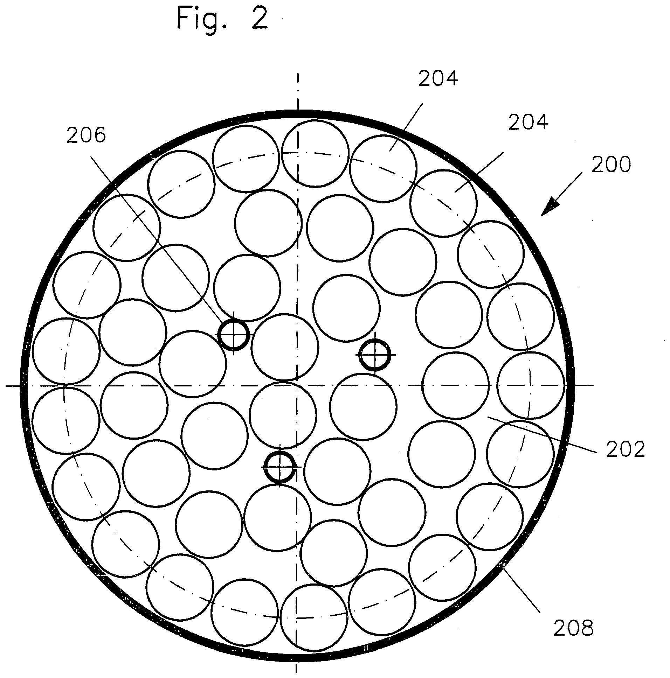

**Figur 2** zeigt beispielhaft eine schematische Darstellung eines Abschnittes einer Nervenprothese 200, welche eine Vielzahl von Leitelementen 204, die eine Kopplungselementaufnahme 202 bilden, drei Kopplungselemente 206 sowie einen Mantel 208 umfaßt. Insbesondere kann Figur 2 eine Schnittansicht von Figur 1 sein, so daß die Nervenprothese 100 und die Nervenprothese 200 identisch sein können, die Leitelemente 104 und die Leitelemente 204 identisch sein können, die Kopplungselemente 106, 106' und die Kopplungselemente 206 identisch sein können und der Mantel 108 und der Mantel 208 identisch sein können.

Es ist aus der Figur 2 ersichtlich, daß die Leitelemente 204 zum Teil ohne bestimmte bzw. erkennbare Regel aneinander angeordnet sein können. Hingegen ist ein anderer Teil (die äußere Reihe der Leitelemente 204) auf einer Symmetrielinie angeordnet.

Die Kopplungselemente 206 sind neben Leitelementen 204 angeordnet. In einer modifizierten Ausführungsform könnte zumindest eines der Kopplungselemente 206 in einem der Leitelemente 204 angeordnet sein. Die Ausführungsform der Nervenprothese 200 der Figur 2 zeigt, daß die Leitelemente 204 und Kopplungselemente 206 jeweils gleich große Durchmesser bzw. Dimensionierungen in eine Querrichtung aufweisen.

In einer modifizierten Ausführungsform können die Leitelemente 204 bzw. die Kopplungselemente 206 in ihrer Dimensionierung zueinander ebenfalls gleich sein.

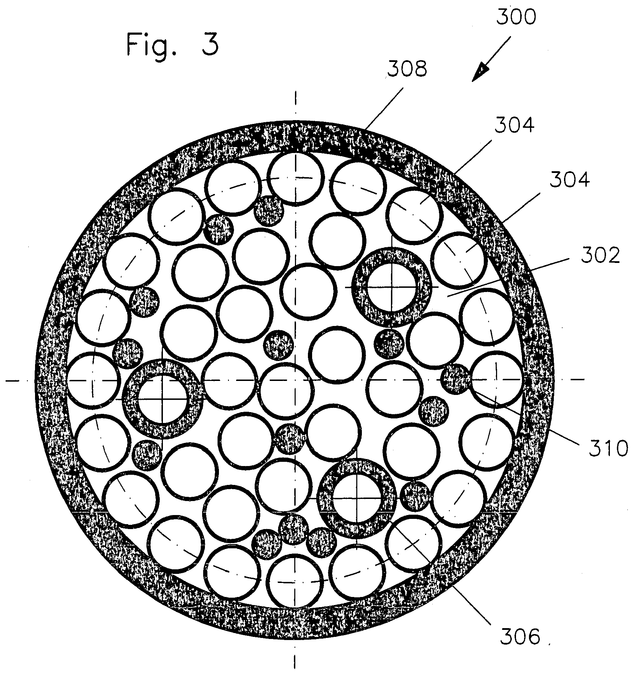

**Figur 3** zeigt eine weitere schematische Darstellung eines Abschnittes einer Nervenprothese 300 im Schnitt entlang einer Querrichtung, welche eine Vielzahl von Leitelementen 304, 310, drei Kopplungselemente 306 sowie einen Mantel 308 umfaßt.

Die Nervenprothese 100 und die Nervenprothese 300 können identisch sein, die Leitelemente 104 und die Leitelemente 304 können identisch sein, die Kopplungselemente 106, 106' und die Kopplungselemente 306 können identisch sein und der Mantel 108 und der Mantel 308 können identisch sein.

Die Leitelemente 304 der Kopplungselementaufnahme 302 sind röhrenförmig ausgebildet, während die Leitelemente 310 der Kopplungselementaufnahme 302 stabförmig ausgebildet sind. Insbesondere können die stabförmigen Leitelemente 310 als stabilisierende Elemente ausgebildet sein, welche eine ausreichende Steifigkeit bzw. Stabilität der Nervenprothese 300 gegenüber äußeren Einwirkungen bereitstellt, während die Leitelemente 304 eher flexibel und biegsam sind, so daß eine Nervenprothese umgebendes Gewebe nicht gequetscht bzw. geschädigt wird, wenn eine Nervenprothese in eine zu behandelnde Körperregion eingesetzt wurde.

Wie die Figur 3 zeigt, sind die Kopplungselemente 306 in ihrer Dimensionierung größer als die röhrenförmigen Leitelemente 304.

Ein Fluidaustausch (wie Blut, Hormone, etc.) kann über die das Kopplungselement umgebenden Leitelemente erzielt bzw. vorgenommen werden. Weiterhin können in einer modifizierten Ausgestaltung die stabilisierenden Elemente wie die in Figur 3 dargestellten Kopplungselemente 306 ausgebildet sein. Durch eine derartige hohlzylindrische Ausgestaltung kann eine höhere Biegefestigkeit eines stabilisierenden Elements im Vergleich zu einem stabförmigen stabilisierenden Element erzielt werden, während das Gewicht genauso wie das Verdrängungsvolumen geringer erhöht sind als bei einer vollzylindrischen Ausgestaltung.

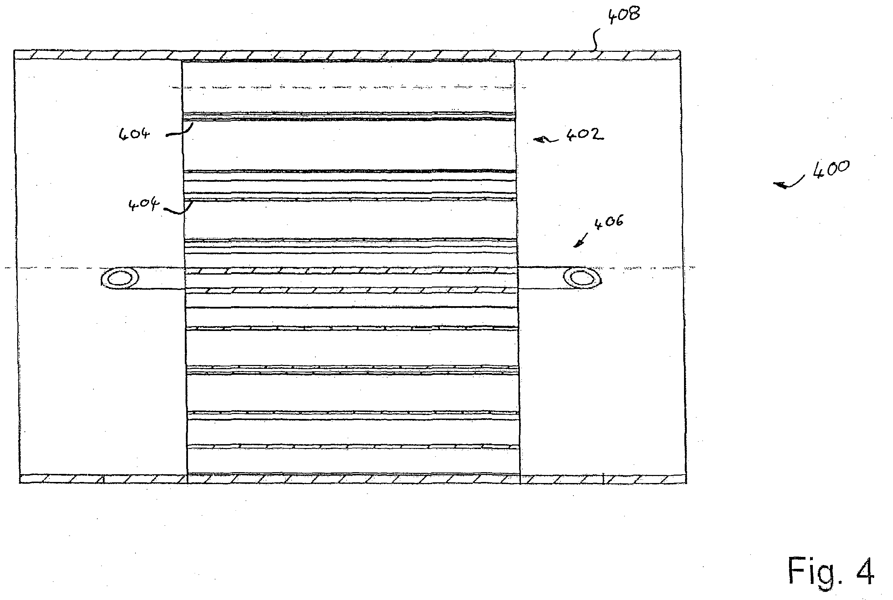

**Figur 4** zeigt eine schematische Darstellung einer Nervenprothese 400 im Schnitt entlang einer Längsrichtung, welche eine Kopplungselementaufnahme 402 mit Vielzahl von Leitelementen 404, ein Kopplungselement 406 sowie einen Mantel 408 umfaßt.

Die Nervenprothese 100 und die Nervenprothese 400 können identisch sein, die Kopplungselementaufnahme 102 und die Kopplungselementaufnahme 402 können identisch sein, die Leitelemente 104 und die Leitelemente 404 können identisch sein, die Kopplungselemente 106, 106' und die Kopplungselemente 406 können identisch sein können und der Mantel 108 und der Mantel 408 können identisch sein.

Beispielsweise kann durch zumindest eines der Leitelemente 404 ein chirurgischer Faden (nicht gezeigt) durchgeführt sein. Weiterhin können mehrere chirurgische Fäden durch ein oder verschiedene Leitelemente 404 geführt sein, welche nach einem Anordnen der Nervenprothese 400 an einen Nervbereich ein Fixieren des Nervbereichs mittels des chirurgischen Fadens bzw. mittels der chirurgischen Fäden erlaubt. Ein Fixieren kann beispielsweise durch ein Umschlingen des Nervbereichs mit dem Faden oder ein Annähen des Nervbereichs erfolgen.

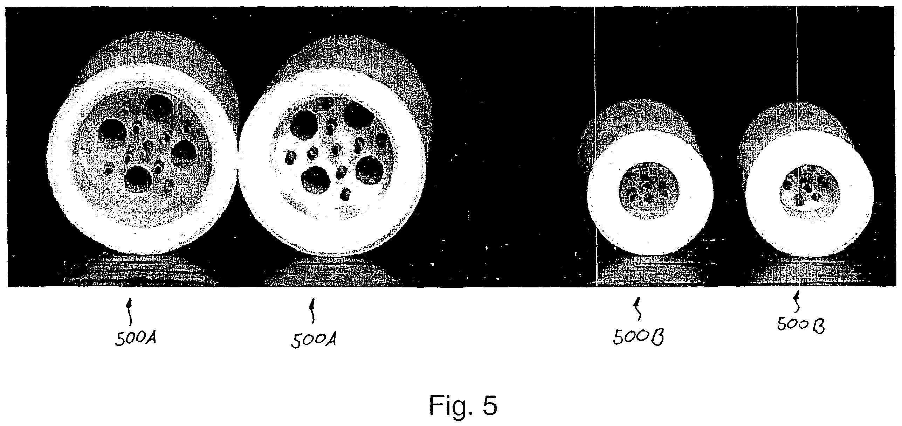

**Figur 5** zeigt eine perspektivische Darstellung einer Nervenprothese 500A sowie einer Nervenprothese 500B.

Die Nervenprothesen 500A und 500B sind jeweils als integrale Element ausgestaltet. Anstatt einer Mehrzahl von Leitelementen weisen die Nervenprothesen 500A und 500B jeweils mehrere Kanäle auf. Insbesondere ist ersichtlich, daß zumindest in einigen Kanälen Kopplungselemente angeordnet sind.

Die Nervenprothese 500A weist neben Kanälen, welche Kopplungselemente umfassen, zusätzlich Kanäle auf, welche im Vergleich größer dimensioniert sind, um ein Durchwachsen von Gewebe zu erleichtern bzw. zu begünstigen.

Wie gezeigt, sind insbesondere Kopplungselementaufnahmen der Nervenprothesen 500A und 500B integral ausgestaltet. Weiterhin kann ein Mantel mit einer Kopplungselementaufnahme integral ausgebildet sein.

In einer modifizierten Ausgestaltung einer Nervenprothese 500A bzw. 500B weist die Nervenprothese keinen Mantel auf. Beispielsweise kann auf einen Mantel verzichtet werden, wenn durch eine integrale Ausgestaltung der Kopplungselementaufnahme eine für den Anwendungsfall ausreichende Stabilität der Nervenprothese gewährleistet ist.

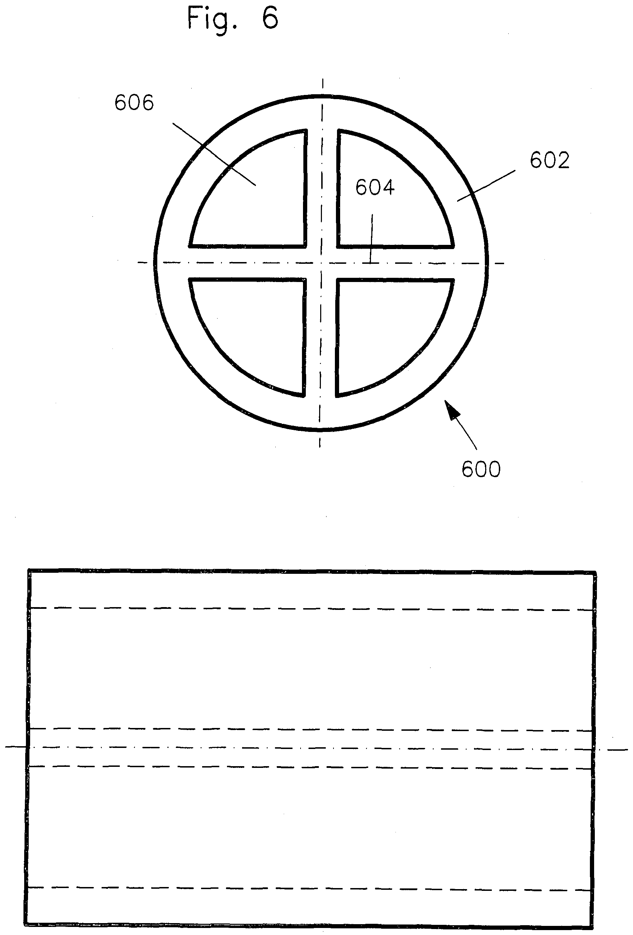

**Figur 6** zeigt eine schematische Darstellung einer Ausgestaltung einer Kopplungselementaufnahme 600 in der Draufsicht, wobei Kopplungselemente in einem Umfangsbereich 602 und/oder in einem Bereich der Verbindungsstreben 604 oder in Zwischenbereichen 606 anordenbar sind.

Weiterhin zeigt Figur 6 eine schematische Darstellung einer Ausgestaltung der Kopplungselementaufnahme 600 in der Seitenansicht.

Die Nervenprothesen 100, 200, 300, 400, 500 gezeigt in den Figuren 1 bis 5 können jeweils Nervenprothesen sein, wobei der Mantel und die Kopplungselementaufnahme resorbierendes Material umfassen, so daß die Nervenprothese nach einer Absorptionszeit im wesentlichen absorbiert ist.

Weiterhin können die Nervenprothesen 100, 200, 300, 400, 500 als das resorbierende Material Gelatine und/oder Polyglycolsäure umfassen.

Zumindest eines der Kopplungselemente der beschriebenen Nervenprothesen 100, 200, 300, 400, 500 kann aus nicht-resorbierbarem Material bestehen. Insbesondere kann das nicht-resorbierbare Material ein Metall sein. Vorzugsweise ist das Metall Titan, ein chirurgischer Stahl oder ein im medizintechnischen Bereich verwendetes Material.

Weiterhin umfassen die Nervenprothesen 100, 200, 300, 400, 500 zumindest einen biochemischen Stimulator, um ein Koppeln von zwei Nervbereichen zu stimulieren, wobei der zumindest eine Stimulator in einem oder mehreren Leitelementen und/oder Kanälen und/oder zumindest einem Kopplungselement angeordnet ist.

Bei Verletzungen bzw. bei der kompletten Durchtrennung von Nerven kommt es zu einer teilweisen bzw. vollständigen Unterbrechung von Nervensignalen, welche in weiterer Folge eine Degeneration der betroffenen Nerven verursacht und infolgedessen zu einer starken Beeinträchtigung der normalen Tätigkeit des Betroffenen führt. Mit Hilfe der Nervenprothesen 100 bis 500 soll die Nervenregeneration verbessert und die Muskelatrophie verhindert, die körpereigenen Reparationsvorgänge beschleunigt und die Signalübertragung über die verletzte Nervenregion vollständig wiederhergestellt werden.

Vorteilhalfterweise sind die Nervenprothesen 100, 200, 300, 400, 500 als adaptive Nervenprothesen ausgebildet und können nach einem Einsetzen zwischen zwei Nervbereichen eine direkte Verbindung zwischen den vorhandenen getrennten Teilen eines Nervs bzw. einer Nervenfaser wieder herstellen. So ermöglichen Nervenprothesen der beschriebenen Art das gezielte Zusammenwachsen einer getrennten Nervenbahn bzw. Nervs, beispielsweise indem Nervenden bzw. Nervbereiche in eine Nervenprothese hineingesteckt werden und in Folge wieder zusammenwachsen. Vorteilhafterweise können dadurch die Nervensignale, welche über eine Nervenprothese weitergeleitet werden, einen peripheren Anteil eines Nervs bzw. von Gewebe innervieren, die Regeneration fördern und eine atrophische Degeneration eines Erfolgsorganes (wie zum Beispiel die Muskulatur) verhindern.

Insbesondere können die Kopplungselementaufnahmen der beschriebenen Art mit einer bestimmten Anzahl von mehr oder weniger großen Leitelementen, welche als Röhrchen ausgebildet sind, versehen sein. Vorzugsweise umfassen Kopplungselementaufnahmen eine größere Anzahl von verschiedenen Leitelementen bzw. Mikroröhrchen, die miteinander mittels eines Klebemittels verklebt sind.

Vorteilhafterweise sind Nervenprothesen der beschriebenen Art im wesentlichen resorbierbar.

Eine Absorptionszeit kann abhängig von Umgebungsbedingungen in einem zu behandelnden Körper sein. Beispielsweise abhängig von Durchblutungsverhältnissen kann es Absorption bis zu neun Monaten betragen. Eine Absorption erfolgt im wesentlichen durch Hydrolyse ohne Hinterlassung von Restmaterialien. Hierdurch kann insbesondere ein Entstehen von reaktiven Fibrosen, welche die Funktion der regenerierenden Nerven negativ beeinflussen können, verhindert werden. Durch entsprechendes resorbierendes Material einer Nervenprothese kann die Absorptionszeit auf eine entsprechende Körperregion eingestellt werden.

Kopplungselementaufnahmen der beschriebenen Art können zusätzlich Kavernen enthalten, die mit Flüssigkeiten, Gelen oder festen Material gefüllt sind, welche Stimuli enthalten, um über biochemische Reaktionen das Zusammenwachsen getrennter Nerven zu stimulieren.

Weiterhin können Nervenprothesen der beschriebenen Art zusätzlich mechanische und/oder elektrische Stimulatoren umfassen, welche an eine Nervenprothese anordenbar und an diese koppelbar sein können. Vorzugsweise kontaktieren mechanische und/oder elektrische Stimulatoren die zu behandelnden Nervbereiche.

### Bezugszeichenliste

- 100: Nervenprothese
- 102: Kopplungselementaufnahme
- 104: Leitelement
- 106, 106': Kopplungselemente
- 106A, 106B: Kopplungsabschnitte
- 108: Mantel
- 110: Durchlaß 110 eines Kopplungselements
- 112, 114: Enden eines Kopplungselements
- 116, 118: Kontaktbereich
- 120, 122: Adaptionsbereich
- KA: Kopplungsachse

- 200, 300, 400, 500A, 500B: Nervenprothese
- 202, 302, 402: Kopplungselementaufnahme
- 204, 304, 404: Leitelement
- 206, 306, 406: Kopplungselemente
- 208, 308, 408: Mantel
- 310: stabförmiges Leitelement

- 600: Kopplungselementaufnahme
- 602: Umfangsbereich
- 604: Verbindungsstreben
- 606: Zwischenbereichen

## Patentansprüche

1. Nervenprothese (100; 200; 300; 400; 500) umfassend:
eine Kopplungselementaufnahme (102, 202, 302, 402, 600) und
zumindest ein Kopplungselement (106, 106', 206, 306, 406), welches einen ersten und einen zweiten Kopplungsabschnitt (106A, 106A', 106B, 106B') aufweist,
wobei:
das zumindest eine Kopplungselement (106, 106', 206, 306, 406) durch die Kopplungselementaufnahme (102, 202, 302, 402, 600) fixiert ist,
der erste Kopplungsabschnitt (106A, 106A') an einem ersten Nervbereich anordenbar ist und der zweite Kopplungsabschnitt (106B, 106B') an einem zweiten Nervbereich anordenbar ist, so daß die beiden Nervbereiche mittels der Nervenprothese koppelbar sind;
wobei die Kopplungselementaufnahme durch eine Mehrzahl parallel zueinander angeordneter stab- und/oder röhrenförmiger Leitelemente gebildet ist;
**dadurch gekennzeichnet, dass**
das Kopplungselement hohlförmig ausgebildet ist, aus nicht-resorbierbarem Material besteht und das nicht-resorbierbare Material ein Metall und/oder eine Metalllegierung ist.

2. Nervenprothese nach Anspruch 1, wobei das zumindest eine Kopplungselement (106, 106', 206, 306, 406) in der Kopplungselementaufnahme (102, 202, 302, 402, 600) angeordnet ist und der erste und der zweite Kopplungsabschnitt (106A, 106A', 106B, 106B') über einen ersten bzw. zweiten Kontaktbereich (116, 118) der Kopplungselementaufnahme (102, 202, 302, 402, 600) hinausragen.

3. Nervenprothese nach Anspruch 2, wobei die Kopplungsabschnitte (106A, 106A', 106B, 106B') um etwa 0,1 µm bis etwa 8 mm oder um etwa 5 mm bis 3 cm aus dem Kontaktbereich (116, 118) der Kopplungselementaufnahme (102, 202, 302, 402, 600) hervorstehen.

4. Nervenprothese nach einem oder mehreren der vorangegangenen Ansprüche, weiters umfassend einen Mantel (108, 208, 308, 408), wobei die Kopplungselementaufnahme (102, 202, 302, 402, 600) und zumindest teilweise das zumindest eine Kopplungselement innerhalb des Mantels (108, 208, 308, 408) angeordnet sind.

5. Nervenprothese nach Anspruch 4, wobei
der Mantel (108, 208, 308, 408) im wesentlichen ein Hohlzylinder ist, wobei der Mantel (108, 208, 308, 408) um etwa 0,1 µm bis etwa 8 mm oder um etwa 2 mm bis etwa 15 mm über die Kontaktbereiche (116, 118) der Kopplungselementaufnahme (102, 202, 302, 402, 600) hinausragt und/oder
Bereiche des Mantels den ersten und den zweiten Kontaktbereich der Kopplungselementaufnahme (102, 202, 302, 402, 600) umfangen und diese Bereiche jeweils einen ersten bzw. einen zweiten Adaptionsbereich (120, 122) festlegen, wobei der jeweilige Adaptionsbereich (120, 122) eine Weite von etwa 0,5 µm bis etwa 2,8 mm oder von etwa 2,5 mm bis etwa 10 mm oder von etwa 2,3 mm bis etwa 15 mm aufweist.

6. Nervenprothese nach einem oder mehreren der vorangegangenen Ansprüche, wobei die Nervenprothese (100; 200; 300; 400; 500) drei oder mehr Kopplungselemente (106, 106', 206, 306, 406) umfaßt.

7. Nervenprothese nach einem oder mehreren der vorangegangenen Ansprüche, wobei der Mantel und die Kopplungselementaufnahme (102, 202, 302, 402, 600) resorbierendes Material umfassen, so daß die Nervenprothese 100; 200; 300; 400; 500) nach einer Absorptionszeit im wesentlichen absorbiert ist.

8. Nervenprothese nach Anspruch 7, wobei das resorbierende Material Gelatine und/oder Polyglycolsäure ist.

9. Nervenprothese nach einem oder mehreren der Ansprüche 1 bis 8, wobei das nicht-resorbierbare Material Titan und/oder chirurgische Stähle umfaßt.

10. Nervenprothese nach einem oder mehreren der vorangegangenen Ansprüche, umfassend zumindest einen biochemischen Stimulator, wobei der biochemische Stimulator ausgelegt ist, ein Koppeln der beiden Nervbereiche zu stimulieren, und wobei der zumindest eine Stimulator in einem oder mehreren der Leitelemente (104, 204, 304, 404) und/oder in einem oder mehreren Kanälen und/oder in dem zumindest einen Kopplungselement (106, 106', 206, 306, 406) angeordnet ist und/oder ein chirurgischer Faden in der Kopplungselementaufnahme (102, 202, 302, 402, 600) angeordnet ist.

11. Nervenprothese nach einem oder mehreren der vorangegangenen Ansprüche, zur Verwendung in der Behandlung von durchtrennten Nerven.

12. Verfahren zum Herstellen einer Nervenprothese, umfassend die Schritte:
- Bereitstellen einer Mehrzahl parallel zueinander angeordneter Leitelemente; und
- Anordnen zumindest eines hohlförmig ausgebildeten Kopplungselements an die Mehrzahl Leitelemente; wobei das zumindest eine Kopplungselement (106, 106', 206, 306, 406) aus nicht-resorbierbarem Material besteht, und wobei das nicht-resobierbare Material ein Metall und/oder eine Metalllegierung ist, und wobei die Mehrzahl Leitelemente (104, 204, 304, 404, 310) eine Kopplungselementaufnahme (102, 202, 302, 402, 600) bildet, und wobei das Kopplungselement (106, 106', 206, 306, 406) mittels der Kopplungselementaufnahme (102, 202, 302, 402, 600) fixiert wird.

13. Verfahren nach Anspruch 12, mit dem weiteren Schritt:
- Anordnen eines Mantels, wobei die Kopplungselementaufnahme (102, 202, 302, 402, 600), das zumindest eine Kopplungselement (106, 106', 206, 306, 406) und der Mantel zumindest teilweise durch Pressen, Schmelzen oder Kleben aneinander fixiert werden.

## Claims

1. A neural prothesis (100; 200; 300; 400; 500) comprising:
a coupling element receptacle (102, 202, 302, 402, 600) and
at least one coupling element (106, 106', 206, 306, 406) which has a first and a second coupling portion (106A, 106A', 106B, 106B'), wherein:
the at least one coupling element (106, 106', 206, 306, 406) is fixed by the coupling element receptacle (102, 202, 302, 402, 600),
the first coupling portion (106A, 106A') can be arranged on a first nerve region and the second coupling portion (106B, 106B') can be arranged on a second nerve region such that the two nerve regions can be coupled by means of the neural prothesis;
wherein the coupling element receptacle is formed by a plurality of rod-shaped and/or tubular guiding elements which are arranged parallel to one another;
**characterized in that** the coupling element has a hollow configuration, consists of non-reabsorbing material and the non-reabsorbing material is a metal and/or a metal alloy.

2. The neural prothesis according to claim 1, wherein the at least one coupling element (106, 106', 206, 306, 406) is arranged in the coupling element receptacle (102, 202, 302, 402, 600) and the first and the second coupling portion (106A, 106A', 106B, 106B') project beyond a first or respectively second contact region (116, 118) of the coupling element receptacle (102, 202, 302, 402, 600).

3. The neural prothesis according to claim 2, wherein the coupling portions (106A, 106A', 106B, 106B') protrude by approximately 0.1 µm to approximately 8 mm or by approximately 5 mm to 3 cm from the contact region (116, 118) of the coupling element receptacle (102, 202, 302, 402, 600).

4. The neural prothesis according to one or more of the preceding claims, further comprising a casing (108, 208, 308, 408), wherein the coupling element receptacle (102, 202, 302, 402, 600) and at least partially the at least one coupling element are arranged within the casing (108, 208, 308, 408).

5. The neural prothesis according to claim 4, wherein the casing (108, 208, 308, 408) is substantially a hollow cylinder, wherein the casing (108, 208, 308, 408) projects by approximately 0.1 µm to approximately 8 mm or by approximately 2 mm to approximately 15 mm beyond the contact regions (116, 118) of the coupling element receptacle (102, 202, 302, 402, 600)
and/or
regions of the casing comprise the first and the second contact region of the coupling element receptacle (102, 202, 302, 402, 600) and these regions each define a first or respectively a second adaptation region (120, 122), wherein the respective adaptation region (120, 122) has a width of approximately 0.5 µm to approximately 2.8 mm or of approximately 2.5 mm to approximately 10 mm or of approximately 2.3 mm to approximately 15 mm.

6. The neural prothesis according to one or more of the preceding claims, wherein the neural prothesis (100; 200; 300; 400; 500) comprises three or more coupling elements (106, 106', 206, 306, 406).

7. The neural prothesis according to one or more of the preceding claims, wherein the casing and the coupling element receptacle (102, 202, 302, 402, 600) comprise reabsorbing material such that the neural prothesis (100; 200; 300; 400; 500) is substantially absorbed following an absorption period.

8. The neural prothesis according to claim 7, wherein the reabsorbing material is gelatin and/or polyglycolic acid.

9. The neural prothesis according to one or more of claims 1 to 8, wherein the non-reabsorbing material comprises titanium and/or surgical steels.

10. The neural prothesis according to one or more of the preceding claims, comprising at least one biochemical stimulator, wherein the biochemical stimulator is designed to stimulate a coupling of the two nerve regions, and wherein the at least one stimulator is arranged in one or more of the guiding elements (104, 204, 304, 404) and/or in one or more channels and/or in the at least one coupling element (106, 106', 206, 306, 406) and/or a surgical thread is arranged in the coupling element receptacle (102, 202, 302, 402, 600).

11. The neural prothesis according to one or more of the preceding claims for use in the treatment of severed nerves.

12. A method for producing a neural prothesis, comprising the steps of:
- providing a plurality of guiding elements arranged parallel to one another; and
- arranging at least one coupling element having a hollow configuration on the plurality of guiding elements; wherein the at least one coupling element (106, 106', 206, 306, 406) consists of non-reabsorbing material, and wherein the non-reabsorbing material is a metal and/or a metal alloy,
and wherein the plurality of guiding elements (104, 204, 304, 404, 310) forms a coupling element receptacle (102, 202, 302, 402, 600), and wherein the coupling element (106, 106', 206, 306, 406) is fixed by means of the coupling element receptacle (102, 202, 302, 402, 600).

13. The method according to claim 12, with the further step of:
- arranging a casing, wherein the coupling element receptacle (102, 202, 302, 402, 600), the at least one coupling element (106, 106', 206, 306, 406) and the casing are at least partially fixed to each other by pressing, melting or bonding.

## Revendications

1. Prothèse nerveuse (100 ; 200 ; 300 ; 400 ; 500) comprenant :
un logement d'élément de raccordement (102, 202, 302, 402, 600) et
au moins un élément de raccordement (106, 106', 206, 306, 406) présentant une première et une seconde section de raccordement (106A, 106A', 106B, 106B'),
dans laquelle :
l'un au moins des éléments de raccordement (106, 106', 206, 306, 406) est fixé par le biais du logement d'élément de raccordement (102, 202, 302, 402, 600), la première section de raccordement (106A, 106A') peut être disposée sur une première zone du nerf et la seconde section de raccordement (106B, 106B') peut être disposée sur une seconde zone du nerf, si bien que les deux zones du nerf sont raccordable à l'aide de la prothèse nerveuse ;
dans laquelle le logement d'élément de raccordement est formé par de multiples éléments conducteurs tubulaires et/ou en forme de barres disposés parallèlement les uns aux autres ;
**caractérisée en ce que** l'élément de raccordement est conçu de manière creuse, qu'il est constitué d'un matériau non résorbable et que le matériau non résorbable est un métal et/ou un alliage métallique.

2. Prothèse nerveuse selon la revendication 1, dans laquelle l'un au moins des éléments de raccordement (106, 106', 206, 306, 406) est disposé dans le logement d'élément de raccordement (102, 202, 302, 402, 600) et que la première et la seconde section de raccordement (106A, 106A', 106B, 106B') dépassent au-dessus d'une première et d'une seconde zone de contact (116, 118) du logement d'élément de raccordement (102, 202, 302, 402, 600).

3. Prothèse nerveuse selon la revendication 2, dans laquelle les sections de raccordement (106A, 106A', 106B, 106B') sont en saillie par rapport à la zone de contact (116, 118) du logement d'élément de raccordement (102, 202, 302, 402, 600) d'environ 0,1 µm jusqu'à environ 8 mm ou d'environ 5 mm jusqu'à 3 cm.

4. Prothèse nerveuse selon l'une ou plusieurs des revendications précédentes, comprenant en outre une gaine (108, 208, 308, 408), dans laquelle le logement d'élément de raccordement (102, 202, 302, 402, 600) et l'un au moins des éléments de raccordement sont disposés en partie au moins à l'intérieur de la gaine (108, 208, 308, 408).

5. Prothèse nerveuse selon la revendication 4, dans laquelle la gaine (108, 208, 308, 408) est essentiellement un cylindre creux, dans laquelle la gaine (108, 208, 308, 408) dépasse au-dessus des zones de contact (116, 118) du logement d'élément de raccordement (102, 202, 302, 402, 600) d'environ 0,1 µm jusqu'à environ 8 mm ou d'environ 2 mm jusqu'à environ 15 mm et/ou
des zones de la gaine entourent la première et la seconde zone de contact du logement d'élément de raccordement (102, 202, 302, 402, 600) et ces zones déterminent respectivement une première et une seconde zone d'adaptation (120, 122), dans laquelle la zone d'adaptation respective (120, 122) présente une largeur d'environ 0,5 µm jusqu'à environ 2,8 mm ou d'environ 2,5 mm jusqu'à environ 10 mm ou d'environ 2,3 mm jusqu'à environ 15 mm.

6. Prothèse nerveuse selon l'une ou plusieurs des revendications précédentes, dans laquelle la prothèse nerveuse (100 ; 200 ; 300 ; 400 ; 500) comporte trois éléments de raccordement (106, 106', 206, 306, 406) ou plus.

7. Prothèse nerveuse selon l'une ou plusieurs des revendications précédentes, dans laquelle la gaine et le logement d'élément de raccordement (102, 202, 302, 402, 600) renferment du matériau résorbable, si bien que la prothèse nerveuse (100 ; 200 ; 300 ; 400 ; 500) est absorbée pour l'essentiel après un temps d'absorption.

8. Prothèse nerveuse selon la revendication 7, dans laquelle le matériau résorbant est de la gélatine et/ou de l'acide polyglycolique.

9. Prothèse nerveuse selon l'une ou plusieurs des revendications précédentes 1 à 8, dans laquelle le matériau non résorbable contient du titane et/ou des aciers chirurgicaux.

10. Prothèse nerveuse selon l'une ou plusieurs des revendications précédentes, comprenant au moins un stimulateur biochimique, dans laquelle le stimulateur biochimique est conçu pour stimuler un raccordement des deux zones du nerf et dans laquelle l'un au moins des stimulateurs est disposé dans un ou plusieurs des éléments conducteurs (104, 204, 304, 404) et/ou dans un ou plusieurs canaux et/ou dans l'un au moins des éléments de raccordement (106, 106', 206, 306, 406) et/ou un fil chirurgical est disposé dans le logement d'élément de raccordement (102, 202, 302, 402, 600).

11. Prothèse nerveuse selon l'une ou plusieurs des revendications précédentes, à utiliser pour soigner des nerfs sectionnés.

12. Procédé de fabrication d'une prothèse nerveuse, comprenant les étapes :
- Mise en place de multiples éléments conducteurs, disposés parallèlement les uns aux autres ; et
- Disposition d'au moins un élément de raccordement de conception creuse près des multiples éléments conducteurs ; dans lequel l'un au moins des éléments de raccordement (106, 106', 206, 306, 406) est constitué d'un matériau non résorbable et dans lequel le matériau non résorbable est du métal et/ou un alliage métallique,
et dans lequel les multiples éléments conducteurs (104, 204, 304, 404, 310) forment un logement d'élément de raccordement (102, 202, 302, 402, 600) et dans lequel l'élément de raccordement (106, 106', 206, 306, 406) est fixé au moyen du logement d'élément de raccordement (102, 202, 302, 402, 600).

13. Procédé selon la revendication 12, présentant l'autre étape :
- Disposition d'une gaine, dans lequel le logement d'élément de raccordement (102, 202, 302, 402, 600), l'un au moins des éléments de raccordement (106, 106', 206, 306, 406) et la gaine sont fixés au moins en partie les uns aux autres par pression, fusion ou collage.
